Europäisches Patentamt

European Patent Office

Office européen des brevets

(19)

(11) **EP 0 261 224 B2**

(12) **NEW EUROPEAN PATENT SPECIFICATION**

(45) Date of publication and mention
of the opposition decision:
**26.03.2003 Bulletin 2003/13**

(45) Mention of the grant of the patent:
**08.12.1993 Bulletin 1993/49**

(21) Application number: **87902905.6**

(22) Date of filing: **20.03.1987**

(51) Int Cl.[7]: **G01N 33/53**, G01N 33/531,
G01N 33/532, G01N 33/543,
G01N 33/571, G01N 33/574,
G01N 33/577, G01N 33/569

(86) International application number:
**PCT/US87/00577**

(87) International publication number:
**WO 87/006005 (08.10.1987 Gazette 1987/22)**

(54) **SYNTHETIC HTLV-III PEPTIDES, COMPOSITIONS AND USES THEREOF**

SYNTHETISCHE HTLV-III-PEPTID-ZUSAMMENSETZUNGEN UND IHRE VERWENDUNG

PEPTIDES SYNTHETIQUES UTILES POUR COMBATTRE L'HTLV-III, COMPOSITIONS ET
UTILISATIONS DESDITS PEPTIDES

(84) Designated Contracting States:
**AT BE CH DE FR GB IT LI LU NL SE**

(30) Priority: **24.03.1986 US 843437**

(43) Date of publication of application:
**30.03.1988 Bulletin 1988/13**

(73) Proprietor: **ORTHO PHARMACEUTICAL
CORPORATION
Raritan New Jersey 08869-0602 (US)**

(72) Inventors:
• **ROSEN, Jonathan, I
San Diego, CA 92131 (US)**
• **NASO, Robert, B.
Carlsbad, CA 92008 (US)**
• **ARLINGHAUS, Ralph, B.
San Diego, CA 92122 (US)**

(74) Representative: **Jones, Alan John et al
CARPMAELS & RANSFORD
43 Bloomsbury Square
London, WC1A 2RA (GB)**

(56) References cited:
EP-A- 0 199 438      EP-A- 0 214 709
WO-A-86/06414      WO-A-87/06005
US-A- 4 134 792      US-A- 4 629 783
US-A- 4 689 398

• A. FETEANU, "Labelled Antibodies in Biology
and Medicine", 1978, Abacus Press (GB); pp.
109-111#
• L. HUDSON et al., "Practical Immunology", 1980,
Blackwell Scientific Publications, London (GB);
pp. 5-9#

• DIAGNOSTIC HORIZONS, vol. 2, no. 1, February
1978, Walkersville, MD (US); A. VOLLER, pp. 1-7#
• NATURE, vol. 313, February 1985, London (GB);
M.A. MUESING et al., pp. 450-458#
• NEW ENGLAND JOURNAL OF MEDICINE, vol.
312, no. 5, January 1985, Boston, MD (US); J.
SCHUPBACH et al., pp. 265-270#
• NATURE, vol. 256, 1975, London (GB); G.
KOHLER et al., pp. 495-497#
• PROCEEDING OF THE NATL. ACADEMY OF
SCIENCES USA, vol. 83, August 1986,
Washington, DC (US); J.J. WANG et al., pp.
6159-6163#
• BIO/TECHNOLOGY, vol. 4, February 1986, The
Internatl. Monthly for Industrial Biology, Nature
Publishing Co., New York, NW (US); C.D.
CABRADILLA et al., pp. 128-133#
• BIO/TECHNOLOGY, vol. 3, October 1985, The
Internatl. Monthly for Industrial Biology, Nature
Publishing Co., New York, NW (US); T.W. CHANG
et al., pp. 905-909#
• Nature 313, 1985, p277-284
• Science 228, 1985, p1091-1094
• Science 228, 1985, p.1094-1096
• Cell 41, 1985, p.979-986
• Science 229, 1985, p.1402-1405
• JP-A-61-30600 (Chemical Abstracts
105,1986,105,Abstract No.173060)
• Das AIDS Virus, Chapter IV-Therapie, 1988,
117-126
• Scientific American, May 1992, 58
• Molecular Immunology 28(6), 1991, 613-622
• J Rosen et al, Cold Spring Harbor Laboratory,
1987, p188-193
• Abstract of papers-"Modern approaches to new
vaccines including prevention of AIDS.
September 9 - September 14 1986. Cold Spring
Harbor Laboratory, p 58

**Description**

FIELD OF THE INVENTION

The subject invention relates to synthetic peptides and, more particularly, to synthetic peptides which mimic a portion of a protein or proteins produced by the HTLV-III or HTLV-III-like viruses that are etiologically associated with the disease syndromes known as AIDS and ARC.

BACKGROUND OF THE INVENTION

Acquired Immune Deficiency Syndrome (AIDS) was first recognized in the United States in 1981, and the number of United States cases has been doubling approximately every ten months since then. Since 1981 there have been about 16,000 reported AIDS cases in the United States, of which approximately half have already died. The expected outcome of the disease is invariably death, since there is currently no known treatment which can effectively delay or prevent the ravages of the disease. Although the disease first manifested itself in homosexual or bisexual males and intravenous drug abusers, it has now spread to others by shared use of contaminated needles or by intimate sexual contact with or receipt of blood products from a carrier of the virus.

The etiological agent associated with AIDS has been identified as a group of related retroviruses variously known as Human T-cell Lymphotropic Viruses-type III (HTLV-III). Human Immunodeficiency Virus (HIV). Lymphoadenopathy Viruses (LAV) or AIDS-Related Viruses (ARV). These viruses will be collectively referred to herein for convenience as "HTLV-III viruses".

Because AIDS can be transmitted by blood products, there has, from the initial recognition of the disease, been a strong impetus to develop diagnostic tests to screen blood for antibodies or antigens specific for the infecting virus. Efforts in this area have borne fruit, and by the end of 1985 five companies had been approved to market tests to detect antibodies to HTLV-III virus. These tests all rely for detection of the antibodies on the use of viral proteins obtained from cultured HTLV-III infected T-lymphocytes. The virus obtained from the cultured cells is disrupted (e.g., with detergent) and a fluid (called "viral lysate") is obtained. This lysate (containing a variety of fragments of viral protein) is then typically used as the solid phase component of an immunoassay.

The current commercial immunoassays are of the conventional sandwich ELISA format, in which the solid phase component (having the viral lysate deposited thereon) is contacted with blood or serum suspected of containing HTLV-III antibodies. If the antibodies are present, they are expected to bind to the viral lysate and, after unbound material is washed away, are contacted with enzyme-labeled anti-human immunoglobulin. The labeled antibodies will bind to any human antibodies attached to the solid phase; the specificity of the test for HTLV-III antibodies is therefore conferred by the viral lysate.

While the existing tests appear to have significantly diminished the transmission of HTLV-III virus via blood products, these tests based on viral lysate have some significant disadvantages.

First, because the viral lysate is produced from cells infected with live HTLV-III virus, there is a possibility that those making the test might be infected during manufacture. There is also at least the theoretical possibility that the live virus might survive the disruption procedure and find its way into the diagnostic test and thus infect the user.

A second disadvantage involves the difficulty of obtaining the lysate and the variable nature of the resulting lysate depending on variation in processing procedure or in characteristics of the infected cells used to obtain the viral lysate.

A third, and far more serious, practical disadvantage is the substantial number of false positive and, to a lesser extent, false negative results observed using the current tests. It is by now well recognized that the current viral lysate ELISA tests yield a substantial number of false positive results. As observed in the Hastings Center Report, Special Supplement/August 1985, entitled "AIDS; The Emerging Ethical Dilemmas":

"However, the ELISA test is not as specific as it is sensitive; that is, in a population of healthy blood donors, as many as one out of every hundred test results will be positive; and from these, as many as 90 out of 100 will be falsely positive. On a national scale, out of the 8 million blood donations each year, 40,000 will be falsely positive." (Page 9)
The false positives are thought to be due in part to the presence of non-viral proteins in the viral lysate preparations used in the solid phase component of the current assays.

The report further indicates that a Western blot confirmatory test (which is more expensive and more technically difficult than the ELISA test) must be performed to exclude the false positive results. Since Western blot assays are themselves subject to error and subjective interpretation, a simple, quick and objective confirmation test for HTLV-III antibody is still desirable.

False negatives are also a concern, although such false negatives may result partially from the nature of the assay, from the immunosuppressive nature of the disease, or from the latency period between exposure to HTLV-III virus and development of antibody.

A further disadvantage of the currently-available tests is that they detect only antibody to the HTLV-III virus and not the virus or viral antigen itself. A positive result therefore indicates only that the tested subject was at one time exposed to the HTLV-III virus and developed antibodies thereto; it is inconclusive about whether the subject is currently infected, or is infectious, or has AIDS. It should be noted that in the revised Center for Disese Control definition of AIDS (June 1985) patients are excluded as AIDS cases if they are negative for serum antibody to HTLV-III and do not have a low number of T-helper lymphocytes or a low ratio of T-helper to T-suppressor lymphocytes.

Despite their inconclusive diagnostic value, the current tests are being used not only to detect virus-contaminated blood, but also to detect infected persons (whom it is assumed can possibly progress to frank AIDS and transmit the disease to others). Because a substantial number of people who are positive in the commercially-available antibody tests neither exhibit clinical symptoms nor (apparently) are capable of infecting others, there is a substantial risk with current tests of falsely identifying a person as an AIDS carrier with the consequent social and psychological results.

Many of the disadvantages of the current lysate-based test could be obviated by substituting for the viral lysate a material not derived from virus-infected cells. Such a material could be, for example, a virus-specific synthetic peptide or a virus-specific peptide derived from a recombinant organism (typically E. Coli).

Work on the latter approach has been reported by Robert C. Gallo and coworkers in a variety of recent articles, including Biotechnology. Vol. 3, Pages 905-909 (October 1985), Science. Vol. 228, Pages 93-96 (April 5, 1985), and Nature, Vol. 315, Pages 151-154 (May 9, 1985), These workers have identified an 82 amino acid peptide encoded by a gene segment in the ENV region of the HTLV-III virus produced through recombinant E. Coli techniques. This peptide is recognized by antibodies to HTLV-III virus. More recently, a Genentech group has reported work on a 102 amino acid peptide containing within it the 82 amino acid peptide of the Gallo group. Biotechnology, Vol.4, Pages 128-133 (February 1986)

While the synthetic peptide technique offers more apparent advantages (e.g., specificity, purity, ease of preparation, and the like) there appears to have been little work in this area. In fact, the only publication made available to the public before the priority date of which the present inventors are aware that relates to such work is a presentation by Dr. Dino Dina and coworkers at the Fifth Annual Congress for Recombinant DNA Research, February 3-6, 1985. While the Abstract of the presentation indicates that various synthetic peptides "are being used (1) to assess immune responses in AIDS patients and (2) to raise antisera in animals", the specific peptides used were not disclosed. Moreover, in the presentation itself, no specific peptides were identified and no indication was given that any one peptide or combination of peptides could recognize all or most of the known positive sera. Subsequently, in August of 1986 a publication by Wang et al., Proc. Natl. Acad. Sci., U.S.A., (1986) 83:6159-6163 was published.

Synthetic peptides which would successfully mimic a portion of the HTLV-III protein and hence would be useful both for detection of antibody to HTLV-III and for production of antibody which would recognize HTLV-III virus or viral antigen would be a significant advance in the art. The present invention provides such peptides, compositions containing such peptides, and methods for using these peptides and compositions for therapy and diagnosis. The invention also provides anti-peptide antibodies, compositions containing these antibodies, and methods for using the antibodies and compositions.

SUMMARY OF THE INVENTION

The present invention provides synthetic peptides, as defined in claims 1 and 2, selected from the group consisting of:

IWGCSGKLICTTAVP    (II)

IWGCSGKLICTTAVPWNAS    (III)

AVERYLKDQQLLGIWGCSGKLI    (IV)

AVERYLKDQQLLGIWGCSGKLICTTAVPWNAS    (V)

LKDQQLLGIWGCSGKLI    (VI)

QQLLGIWGCSGKLICTTAVPWNAS     (VIII)

CSGKLICTTAVPWNAS     (X)

AVERYLKDQQLLGIWGCSGKLIC     (XII)

GCSGKLICTTAVPWN     (XIII)

LKDQQLLGIWGCSGK     (XIV)

These peptides are recognized by a majority of HTLV-III antibody-positive sera from patients with AIDS/ARC, as well as antibody-positive sera of unknown diagnosis. In addition, the subject peptides yield hardly any false positives.

These results are highly surprising in view of the fact that other peptides corresponding to portions of the HTLV-III envelope proteins are ineffective to selectively recognize antibodies to HTLV-III virus.

The present invention further comprises the discovery that recognition of antibodies to HTLV-III virus is significantly enhanced if certain peptides as defined in claim 3 are used in combination.

The combination of peptides (IV) or (XII) with peptides (III), (XIII), or (X) are especially preferred. The combination of (III) and (IV) or (XIII) and (IV) being the combinations of choice.

In view of these results, it is clear that a significant antigenic determinant of the HTLV-III virus which reacts with HTLV-III antibodies is contained within the seven amino acid residue peptides that include the sequence of formula CSGKLIC (I). Moreover, even though each of the subject peptides reacts with most HTLV-III positive sera, individual patient sera have been observed to react specifically with one of the subject peptides but not another. This observation indicates that additional antigenic determinants exist in longer peptides containing the sequence of formula (I), such as peptides (II) through (X).

The peptides of the invention contain at least one cysteine residue, and in certain instances two of such residues. Accordingly, the subject peptides may exist in various oxidative forms. In addition to the monomeric form-in which the sulfhydryl group of the cysteine residue(s) is reduced, there may also exist dimeric-or-polymeric forms-in which sulfhydryl groups on two or more peptide molecules become oxidized and form disulfide bonds. While subject peptides that possess only one cysteine residue can form only linear dimers, those that possess two cysteine residues may form cyclic monomers or linear or cyclic dimers and linear polymers of various lengths. These various oxidative forms are considered part of the subject invention are are included in the terms "subject peptides".

The present invention further provides a method of detecting or determining HTLV-III antibodies and a diagnostic kit for detection or determination of HTLV-III antibodies using one or more synthetic peptides of the invention. The subject method for detecting or determining antibodies to HTLV-III in a fluid suspected of containing said antibodies e.g. blood, serum, plasma, saliva or urine, is defined in claim 8.

It may be useful to separate the solid surface from said fluid and to wash unbound material from the solid surface material after step b, depending on the detection method employed. While the specific detection technique is not critical, enzyme labelled anti-human immunoglobulin has been found to perform well. The subject diagnostic kit for practicing this method comprises a solid surface having at least one subject peptide bound thereto and labelled (preferably enzyme labelled) anti-human immunoglobulin. Other conventional materials for labelling antibody may also be used, as for example, biotin or a radioisotope.

In addition, the present invention provides a method of preparing anti-peptide antibodies useful for detection of HTLV-III virus or viral antigen which comprises immunizing a host animal with a subject peptide in polymerized form or attached to a suitable immunogenic carrier. The method for preparing polyclonal antibodies to HTLV-III comprises immunizing a host animal with at least one subject peptide in polymerized form or attached to a suitable immunogenic carrier and bleeding the host. The method for preparing monoclonal antibodies to HTLV-III comprises immunizing a host animal with at least one subject peptide in polymerized form or attached to a suitable immunogenic carrier, isolating the splenocytes from said immunized host, fusing said splenocytes with a suitable myeloma cell line, selecting the fused cells by reactivity to the immunizing peptide or fragment, to HTLV-III, or to HTLV-III-infected cells, and either culturing the selected hybridoma in vitro or injecting it into a suitable host. The resulting desired monoclonal antibody may be recovered from the supernatant over the cultured hybridoma or from the serum or ascites of the innoculated host.

The antipeptide antibodies themselves, the method of detecting HTLV-III virus using the antibodies. and diagnostic kits containing said antibody (useful for detecting HTLV-III virus) are also included within the subject invention.

A sandwich method according to the invention is defined is claim 12.

It may be useful to separate the solid surface from the fluid to be tested and to wash away unbound material from the solid surface after step b, depending on the detection method employed.

A competition method according to the invention is defined in claim 13.

A diagnostic kit for detecting or determining HTLV-III virus by sandwich or competition method is defined in claim 14.

A subject peptide may also be used to improve the specificity of the subject peptide test and thus reduce reliance on the more difficult Western blot confirmatory test. The accuracy of a positive result in the subject peptide test may be confirmed by repeating the test in the presence of an effective blocking amount of a subject peptide If binding of the supposed HTLV-III antibodies to the plate is blocked by the added peptide, the original positive result is confirmed; if binding is not blocked then the original result was a false (non-specific) positive.

The present invention therefore also includes a method of determining the accuracy of a positive result of a test for HTLV-III antibodies on a fluid sample suspected of containing said antibodies, said test being a sandwich assay in which a synthetic HTLV-III peptide is attached to the solid surface, which method is defined in claim 15.

The phrase "effective blocking amount" of the subject peptide means an amount which will substantially completely react with any HTLV-III antibodies directed against the antigenic determinant(s) contained in said peptide that are present in the tested sample and thus substantially block reaction of these antibodies with the peptide on the solid surface of the assay. The sandwich assay is preferably an ELISA assay.

The subject peptides may be prepared by any conventional technique (including 'DNA technology and liquid phase synthetic techniques), although solid-phase Merrifield-type synthesis is a convenient way of preparing and isolating the peptide. A further description of this technique and of other art-known techniques may be found in the literature, i.e.. M. Bodanszky, et al., Peptide Synthesis, John Wiley & Sons, Second Edition, 1976, as well as in other reference works known to those skilled in the art. Synthetic techniques (as opposed to 'DNA techniques) are preferred for reasons of purity, antigenic specificity, freedom from undesired side products, ease of production, and the like. Appropriate protective groups usable in such syntheses and their abbreviations will be found in the above text, as well as in J.F.W. McOmie, Protective Groups in Organic Chemistry, Plenum Press, New York, 1973. Both of these books are incorporated herein by reference.

Typical carriers to which the subject peptides may be attached for generation of anti-peptide antibodies include, e.g., bovine serum albumin; tetanus toxoid: keyhole limpit hemacyanin: porcine, bovine or equine immunoglobulin, and cholera or E. coli heat-labile toxin B-subunit.

The structures or the subject peptides are given in the conventional single-letter codes for amino acids and are to be read, from left to right, as directionally corresponding to the amino to carboxy direction of the sequence. For the convenience of the reader, the single-letter codes for the amino acids contained in the subject peptides are: I = L-isolucine; W = L-tryptophan; G = glycine; C = L-cysteine; S = L-serine; K = L-lysine; L = L-leucine: T=L-threonine; A = L-alanine; V = L-valine; P=L-proline; D = L-aspartic acid; N = L-asparagine; E=L-glutamic acid; R = L-arginine; Y = L-tyrosine; Q = L-glutamine; M = L-methionine; and F = L-phenylalanine.

The present invention is illustrated by the following examples.

EXAMPLE I

A. Synthesis of BOC-Proline Resin:

Chloromethylated styrene-divinylbenzene polymer containing 1.3 meq chloride/gram of resin was esterified with Boc-proline in anhydrous N,N-Dimethylformamide (DMF) using potassium iodide (KI) as the catalytic agent. The reaction was done at 55°C for 24 hours (1). The substitution of Boc-Proline was 0.92 mMole gram as determined using a picric acid assay on a portion of deblocked resin.

B. Synthesis and Characterization of Peptide (II):

Synthesis of the peptide of formula (II) was accomplished using classical Mernfield technique (2). The peptide sequence 'IWGCSGKLICTTAVP' was synthesized on a Vega 250C automated peptide synethsizer using a double couple program. 1.8326g of Boc-proline resin was sequentially double coupled with the following Boc-L-amino acids (3.8) in twelve meq excess:

| Amino Acid | Solvent |
|---|---|
| Boc-Val | $CH_2Cl_2$ |
| Boc-Ala | $CH_2Cl_2$ |
| Boc-(O-Bzl)-Thr | $CH_2Cl_2$ |
| Boc-(O-Bzl)-Thr | $CH_2Cl_2$ |
| Boc-(MeOBzl)-Cys | $CH_2Cl_2$ |
| Boc-Ile | $CH_2Cl_2$ |
| Boc-Leu | 10% DMF $CH_2Cl_2$ |
| Boc-(Cl-Z)-Lys | $CH_2Cl_2$ |
| Boc-Gly | $CH_2Cl_2$ |
| Boc-(O-Bzl)-Ser | $CH_2Cl_2$ |
| Boc-(MeOBzl)-Cys | $CH_2Cl_2$ |
| Boc-Gly | $CH_2Cl_2$ |
| Boc-Trp | 10% DMF $CH_2Cl_2$ |
| Boc-Ile | $CH_2Cl_2$ |

The peptide was cleaved from the resin with 10% anisole in hydrofluoric acid and extracted with 20% aqueous acetic acid. This solution was filtered to remove solid resin and run through a Fractogel TSK HW-40F desalting column using an eluent of 20% aqueous acetic acid. Fractions were collected in 10 ml aliquots and the column effluent monitored at 280nm. Fractions showing positive absorbance at 280nm were diluted with 0.1% trifluoroacetic acid (TFA) in water and analyzed by high performance liquid chromatography (HPLC)(4). The major peak of absorbance at 214 nm was determined to have a retention time of 12.42 min. The Fractogel fractions that contained greater than 70% of this peak were pooled and labelled Fr:1. The Fractogel fractions that contained less than 70% but greater than 50% of this peak were pooled and labelled FR:2.

Analytical HPLC (4) on Fr:1 showed the 12.42 min peak to constitute 87% of the total area. Fr:1 was further characterized by amino acid analysis (5), sequence determination (6), and determination of % peptide content (7). Peptide sequence analysis (6) was also performed on the peptide-resin to confirm the expected sequence of the peptide.

(1) Stewart & Young, Solid Phase Peptide Synthesis, 2nd edition (1984), Pierce Chemical Co.
(2) Merrifield, R.B. (1963), J. Amer. Chem. Soc. 85, pp. 2149-2154
(3) All Boc-amino acids obtained from Bachem Inc., Torrance, Calif.
(4) Analytical HPLC Conditions:

Buffer A: 0.1% TFA/Distilled Deionized Water
Buffer B: 0.1% TFA/HPLC grade Acetonitrile
Gradient Conditions: 10% 'B' to 50% 'B' over 20 minutes
Wavelength: 214nm
Flow: 1.0 ml/min
Column: Vydac 214TP54 C-4 Protein column, 250 x 4.5 mm

(5) Amino Acid analysis performed on a LKB 4150 ALpha Amino Acid Analyzer.
(6) Peptide sequence determination performed on an Applied Biosystems 470A Protein Sequencer.
(7) Peptide content determined from recovery on amino acid analysis of known amount of peptide.
(8) Boc is a chemical abbreviation for the tert-Butyloxcarbonyl alpha-amino protecting group. The functional group is removed by hydrolysis in 50% Triflouroacetic acid (TFA)/50% Dichloromethane ($CH_2Cl_2$) after the amino acid has been coupled to the growing peptide chain. This action exposes the amino terminus of the chain to allow the next amino acid to be effectively coupled.

In addition to the Boc protecting group on every amino acid, the side chains of some amino acids are further protected from attack by the chemistry of peptide synthesis. These protecting groups, listed in parenthesis on the accounting list, are all stable to the conditions of peptide synthesis, yet are easily removed from the amino acid during cleavage in hydrofluoric acid. Anisole (methylphenyl ether) acts as a nucleophilic scavenger during the HF cleavage step to prevent alkylation of the peptide by the liberated protecting group carbonium ions. The protecting groups for the amino acids listed are defined below:

O-Benzyl: Benzyl-ester; attached to the hydroxyl side chain of both serine and threonine to prevent the acylation or branching of the peptide chain.

MeObzl: 4-Methoxybenzyl; attached to the sulfhydryl group of cysteine to prevent its oxidation during peptide synthesis.

Cl-Z: 2-chlorobenzyloxycarbonyl; attached to the alpha-amino group of lysine to prevent the formation of side chain growth from this site on the peptide.

C. Polymerization of the Peptide of formula (II):

The peptide contained in the Fr:1 pool of part B was lyophilized to remove acetic acid and solubilized at 200 µgm/ml in 0.1 M sodium bicarbonate buffer pH 9.0. Aliquots of this peptide diluted to 20 µgm/ml in sodium bicarbonate buffer were used to coat microtiter wells for ELISAs shown in Table IV. For tests shown below in Tables I-III peptide was solubilized in water at 10 mg/ml and diluted in phosphate buffer pH 7.3 to 5 µgm/ml for coating microtiter wells. The peptide in Fr:1 exists primarily in a single form that is believed to be unoxidized monomer. Because the peptide of formula (II) contains two cysteines, however, it polymerizes upon solubilization in neutral or basic aqueous buffer. The peptide used in ELISAs described below is a mixture of very small amounts of linear monomer, and larger amounts of cyclic monomer (formed by intramolecular disulfide bonding) and even larger amounts of polymers (formed by intermolecular disulfide bonding) of various sizes. Without wishing to be bound thereby, Applicants believe that the polymer forms are important for the reactivities described herein. The cyclic monomer form, while retaining a portion of the antigenicity of the polymer form, is believed to be less efficient in binding to the microtiter wells and is less suited as the solid phase component of the ELISA. The presumed cyclic monomer is revealed as a sharp peak at about 12.7 min retention time in HPLC analysis while the polymer is characterized as a broad peak at approximately 15.9 min retention time. Oxidation conditions may be altered with respect to temperature, pH, peptide concentration, and the like as known to those skilled in the art to alter the proportion of monomer, cyclic monomer and polymer remaining in the preparation, or the size of polymers formed. Small amounts of so called deletion peptides (lacking one or more amino acids) and their oxidation forms may also be found in the peptide preparations used in the ELISA, but these minor impurities do not affect the use of the peptide.

EXAMPLE II

Synthesis and Characterization of Peptides (III) through (XVI):

Synthesis of these peptides was accomplished using substantially the same classical Merrifield technique as described earlier for peptide (II). For peptide (III), Boc-serine resin with substitution of 0.92 mMole/gram was used. For peptide (IV), Boc-isoleucine resin with substitution of 0.8 mMole/gram was used. Synthesis of the Boc-serine and Boc-isoleucine resins was accomplished by the Gisin method as described by Stewart & Young (1).

A. For peptide (III) the peptide sequence 'IWGCSGKLICTTAVPWNAS' was synthesized using the following Boc-L-amino acids in twelve meq excess:

EP 0 261 224 B2

| Amino Acid | Solvent |
|---|---|
| Boc-Ala | $CH_2Cl_2$ |
| Boc-Asn/Hobt | DMF |
| Boc-Trp | 10% DMF/$CH_2Cl_2$ |
| Boc-Pro | $CH_2Cl_2$ |
| Boc-Val | $CH_2Cl_2$ |
| Boc-Ala | $CH_2Cl_2$ |
| Boc-(0-Bzl)-Thr | $CH_2Cl_2$ |
| Boc-(0-Bzl)-Thr | $CH_2Cl_2$ |
| Boc-(MeOBzl)-Cys | $CH_2Cl_2$ |
| Boc-Ile | $CH_2Cl_2$ |
| Boc-Leu | 10% DMF/$CH_2Cl_2$ |
| Boc-(Cl-Z)-Lys | $CH_2Cl_2$ |
| Boc-Gly | $CH_2Cl_2$ |
| Boc-(O-Bzl)-Ser | $CH_2Cl_2$ |
| Boc-(MeOBzl)-Cys | $CH_2Cl_2$ |
| Boc-Gly | $CH_2Cl_2$ |
| Boc-Trp | 10% DMF/$CH_2Cl_2$ |
| Boc-Ile | $CH_2Cl_2$ |

B. For peptide (IV) the peptide sequence 'AVERYLKDQQLLGIWGCSGKLI' was synthesized using the following Boc-amino acids in 12 meq excess:

| Amino Acid | Solvent |
|---|---|
| Boc-Leu | 10% DMF/$CH_2Cl_2$ |
| Boc-(Cl-Z)-Lys | $CH_2Cl_2$ |
| Boc-Gly | $CH_2Cl_2$ |
| Boc-(0-Bzl)-Ser | $CH_2Cl_2$ |
| Boc-(MeOBzl)-Cys | $CH_2Cl_2$ |
| Boc-Gly | $CH_2Cl_2$ |
| Boc-Trp | 10% DMF/$CH_2Cl_2$ |
| Boc-Ile | $CH_2Cl_2$ |
| Boc-Gly | $CH_2Cl_2$ |
| Boc-Leu | 10% DMF/$CH_2Cl_2$ |
| Boc-Leu | 10% DMF/$CH_2Cl_2$ |
| Boc-Gln/Hobt | DMF |
| Boc-Gln/Hobt | DMF |
| Boc-(Bzl)-Asp | $CH_2Cl_2$ |
| Boc-(Cl-Z)-Lys | $CH_2Cl_2$ |
| Boc-Leu | 10% DMF/$CH_2Cl_2$ |
| Boc-(Br-Z)-Tyr | $CH_2Cl_2$ |
| Boc-(Tosyl)-Arg | 10% DMF/$CH_2Cl_2$ |
| Boc-(Bzl)-Glu | $CH_2Cl_2$ |
| Boc-Val | $CH_2Cl_2$ |
| Boc-Ala | $CH_2Cl_2$ |

As with peptide (II), in addition to the Boc protecting group on every amino acid the side chains of some amino acids are further protected from attack by the chemistry of peptide synthesis. In addition to those protecting groups described for the amino acids in the peptide (II) synthesis, the following protecting groups for the amino acids unique to peptides (III) and (IV) were used:

Hobt: 1-hydroxybenzotriazole; used in equimolar amounts to glutamine and asparagine during coupling to prevent dehydration to the nitrile forms.

8

Tosyl: p-toluene sulfonyl; used to acylate the guanidine group in the side chain of arginine.

Bzl: beta-benzyl ester; blocks the carboxyl groups in the side chain of aspartic acid and glutamic acid.

BrZ: 2-bromobenzyloxycarbonyl; blocks the hydroxyl group in the side chain of tyrosine.

Peptides were cleaved from the resin, filtered, extracted with acetic acid and run through a Fractogel desalting column as in Example I. For peptide (IV), Fractogel fractions were analyzed by analytical HPLC and fractions containing at least 30% of the total absorption at 214 nm as the major peak migrating at approximately 14 minutes retention time were pooled. The pooled fractions were chromatographed on carboxymethyl cellulose equilibrated with 0.01 M ammonium acetate, pH 4.4. The column was eluted with a step gradient of ammonium acetate and the fraction eluting at 0.2M ammonium acetate was collected, lyophilized, and analyzed by analytical HPLC. The major peak migrating at 14 minutes retention time comprised between 30% and 40% of the total absorption at 214 nm and the material had an acceptable amino acid content. This material was resolubilized and used in ELISA as described for peptide (II).

For peptide (III), Fractogel fractions were likewise analyzed by analytical HPLC. Fractions containing at least 70% of the total absorption at 214 nm as the major peak migrating at approximately 12.99 minutes retention time were pooled, lyophilized, and analyzed by HPLC and for amino acid content. This material was resolubilized and used in ELISA as described for peptide (II).

When used in combination as the solid phase component in an ELISA, 1 microgram each of peptides (III) and 0.5 micrograms of peptide (IV) were used per microtiter well. The peptide was either dried onto the well at 37°C or "wet packed" onto the plate by incubation overnight at 4°C.

C. For peptide (V), Boc-serine resin was used as described for synthesis of peptide (III). Synthesis of (V) proceeded as described for synthesis of (III) through the addition of the C-terminal isoleucine of peptide (III). From that point on, for completion of the (V) sequence, the procedure for addition of the amino acids in the sequence AVERYLKDQQLLG in peptide (IV) was followed.

Peptide (V) was cleaved from the resin, filtered, extracted with acetic acid and run through a Fractogel desalting column as described in Example I. Fractogel fractions containing the major peak of absorption at 280 nm were pooled and labelled Fr:1. Fr:1 was analyzed for amino acid content and found to be acceptable. This fraction was lyophilized and used in ELISA as described for peptide (II).

D. Following similar procedures the peptides of formulas (I), (VI) through (XVI) were prepared.

E. Polymerization of Peptides (III), (IV), and (V):

Peptides (I) through (III), (V), and (VII) through (X) contain two cysteines. Accordingly, these peptides can polymerize and cyclize through oxidative disulfide bonding. The addition of the four amino acids at the C-terminal end of (III) apparently allows the cyclic form of the peptide to bind to the plastic in the ELISA assay. As a result, the cyclic form of (III) is more effective in solid phase ELISA than is (II) cyclic. The forms of the (II), (III) and (V) peptides used thus far in ELISA to test for HTLV-III antibody recognition have been typically a mixture of linear monomer, cyclic monomer, dimer and polymer. Peptides (IV), (VI) and (XI) contain only one cysteine. These peptides can form a dimer structure through disulfide bonding. Under conditions of solubilization of peptides in preparation for ELISA (e.g., 0.1M sodium bicarbonate buffer pH 9.0) most of the sulfhydryl groups of peptides (II), (III), (IV), and (V) have been converted to the disulfide form.

EXAMPLE III

Preparation of Comparative Peptides

Following similar procedures to that of Examples II and III, for comparative purposes peptides having the following formulae were synthesized:

QLQARILAVERY    (C-I),

AVERYLKDQQLLG    (C-II),

LKDQQLLGIWGCS    (C-III),

IWGCSGKLI    (C-IV),

, and

LICTTAVPWNASWSN    (C-VIII).

These peptides each have sequences corresponding to the sequence of the HTLV-III envelope but fail to conform to the teachings of this invention. Specifically, peptides having the formula (C-I) and (C-II) are sequences upstream from the amino end of the sequence of formula (I) i.e., CSGKLIC. Peptides having the formula (C-III) contain only the amino terminal portion of the sequence of formula (I). Peptides having the formula (C-IV) contain the full sequence of formula (I), but for the carboxy terminal L.-cysteine residue. Peptides having the formula C-VIII are sequences downstream from the carboxy end of the sequence of formula (I), as will be described herein, each of these peptides fails to exhibit the desirable immunoreactive properties.

EXAMPLE IV

Preparation of ELISA Assay Kit and Procedure for Use:

Procedure #1 for ELISA: (Table IV)

(1) Coat ELISA plate with peptide- 1µg/50µl/well in 0.1M NaHCO3 pH9.
(2) Let plates dry overnight uncovered at 37°C; then wash with PBS.
(3) Block plates with 300µl/well of 5%NCS-PBS for 2 hrs. at 37°C.
(4) Shake out blocking buffer and drain well.
(5) Add 50µl/well test antisera for 30 to 120 minutes at 37°C. (If the antisera is to be diluted, use T-wash.) We have used 1:2 to 1:100 dilutions.
(6) Shake out test antisera and wash plate six times with PBS-Tween20. (Tween is a registered trade mark.)
(7) Add 100µl/well 2nd antibody diluted 1:4000 with T-wash. 30 to 120 minutes at 37°C.
(8) Shake out 2nd antibody and wash plate six times with PBS-Tween20.
(9) Add 100µl/well OPD substrate (or 5 µl ABTS solution) for 20 minutes at RT.
(10) Add 50µl/well of 4N H2SO4 to stop OPD reaction (or 100 µl/well of 1% SDS for ABTS reaction).
(11) Read plate on an MR 600 Microplate ELISA plate reader. (490nm for OPD or 405 nm for ABTS)

Reagents:

(1) 0.1M NaHCO3 pH9.
(2) PBS + 5% Normal Calf Serum.
(3) T-wash : (780ml TBS + 20ml NCS + 1.6gm BSA + 0.4ml Tween20) TBS 12.11g Tris Base
    17.5g NaCl
    1800ml H2O
    pH to 7.6 with HCl (Ca.3N)
    final volume at 2000ml
(4) Washing buffer / PBS-Tween20: 0.5ml Tween10/1L PBS.
(5) OPD substrate: 1OPD tablet/3ml H2O/1.24µl 30%$H_2O_2$.
(6) 4N $H_2SO_4$.
(7) ABTS: $H_2O_2$ in 1:1 ratio by volume of solutions supplied by Kirkegaard and Perry Laboratories, Inc., Gaithersbury, Md.)
    ABTS = 2.2'-azino-di-[3-ethyl-benzthiazoline sulfonate]
(8)1% SDS.

The material used in step 1 for coating the ELISA plate is peptide of formula (II), (III), (IV), (V), or a mixture thereof as described above. The second antibody is either a commercially-available peroxidase-labelled, polyclonal antibody (Cappel Laboratories Catalog No. 3201-0231; Peroxidise-conjugated IgG fraction of goat anti-human immunoglobulins) or a peroxidase-labelled mouse monoclonal anti-human IgG antibody, or a mixture of peroxidase-labelled mouse monoclonal anti-human IgG. IgA and IgM antibodies.

Procedure #2 for ELISA (Tables I-III):

(1). Coat ELISA plate with peptides- μg peptide (IV), 0.5 μg peptide (III) 200μl/well in 0.1M carbonate buffer, pH 9.6.
(2). Incubate plates overnight at 4°C.
(3). Block plates with 300 μl/well 1.0% BSA-PBS plus additives for 2 hr at 37°C.
(4). Shake out blocking buffer.
(5). Dry plates for 1.5 hr at 37°C.
(6). Add 200 μl/well 1% Bovine Gamma Globulin - 5% BSA - 0.5% Tween-PBS, pH 7.2.
(7). Add 10 μl/well test sera, incubate 30 min at 37°C.
(8). Shake out test sera, wash plate 5x with PBS-0.5% Tween.
(9). Add 200 μl/well monoclonal anti-human IgG diluted 1:3500 with 50% fetal calf serum-1% horse serum-0.5% Tween-PBS.
(10). Incubate 30 min at 37°C.
(11). Shake out test sera, wash plate 5x with PBS-0.05% Tween.
(12.) Add 200 μl/well OPD substrate and incubate for 30 min at room temperature.
(13). Add 50 μl/well 4 N $H_2SO_4$ to stop reaction.
(14.) Read plate at 490 nm in MR 600 Microplate Reader.

Reagents:

(1). Phosphate Buffered Saline (PBS) pH 7.3

    8.0 gm of sodium chloride
    0.2 gm of potassium phosphate, monobasic
    1.16 gm of sodium phosphate, dibasic
    0.2 gm of potassium chloride
    0.2 gm of thimerosal
    water to 800 ml and mix
    adjust pH if necessary, add water to 1 L

(2). Coating Buffer, pH 9.6

    0.01 M carbonate buffer.

(3). Blocking buffer (1% BSA-PBS plus additives)

    1% Bovine Serum Albumin (Sigma #A7030)
    10 Kμ/ml Aprotinin
    10μg/ml trypsin inhibitor
    10 nM EACA (E-amino caproic acid)
    0.5 mM PMSF (phenyl-methyl-sulfonyl flouride)
    2.0 mM EDTA
    10% glycerol

(4). Specimen Diluent

    10.0 gm Bovine Gamma Globulin, Fraction II, lyophilized
    50.0 gm Bovine Albumin, Fraction V
    0.5 ml Polysorbate 20 (Tween 20)
    Add water to 1 L and mix
    Filter through 0.2 micron filter.

(5). Conjugate Diluent

490 ml PBS
500 ml heat inactivated fetal bovine serum
10 ml heat inactiviated horse serum
0.1 g thimerosal
0.329 g potassium ferricyanide
water to 1 L. mix, filter through 0.2 micron filter

EXAMPLE V

The ELISA kits described in Example IV-procedure 1 made with peptide (II) were evaluated against a panel of sera comprising sera from normal subjects, patients with disorders or diseases unrelated to AIDS, known AIDS patients, known ARC patients, and patients whose diagnosis is unknown but who are antibody positive by commercial tests or by Western blot assay. The results are summarized in Tables I - IV. For comparison, these same sera samples were assayed with commercially available kits and by Western blot assay. The commercial kits selected for these studies were from Abbott Laboratories, North Chicago, III. and Electro-Neucleonics, Inc. (ENI), Columbia, Md.

A. Table 1 shows results with normal sera.

TABLE I

| Assay of Normal Sera by ELISA using peptide (II). | | | | |
|---|---|---|---|---|
| Number of Samples | E8 Assay | ENI Assay | Abbott Assay | Diagnosis/Sample I.D. |
| 198 | - | 5-,194NT | 41-,156NT | Normal |
| 1 | - | - | + | Normal/749 |
| 1 | + | NT | NT | Normal/2846 |
| $\overline{200}$ | | | | |
| Mean of Normals = 0.016 for E8 assay<br>Standard Deviation (S.D.) from themean = 0.017<br>Cutoff Value at Mean + S.D. = 0.104<br>False Positive rate in 200 samples at 0.104 cutoff = 0.5% (1/200)<br>NT = Not Tested | | | | |

As is shown in the above table, subject sera not containing antibodies to HTLV III generally do not react to peptide (II) in a standard ELISA. This allows for calculation of an absorption cutoff value to distinguish between antibody negative and antibody positive sera. In the above assay of 200 normal sera, a cutoff value of 0.104 was selected. At this cutoff the false positive rate is expected to be less than or equal to 0.5%.
B. To indicate the superior effectiveness for eliminating false positives by employing the peptide of formula (II) over the commercially available kits using viral lysate, a number of sera from patients with two disorders unrelated to AIDS, namely Naso-Pharyngeal Carcinoma and Rheumatoid Arthritis, were tested by the subject assay and commercial tests. The results are summarized in Table II below and indicate the increased specificity of the subject assay. Many samples which gave false positive results with commercial tests correctly identified as negative by the subject assay.

TABLE II

| Test of Naso-Pharyngeal Carcinoma and Rheumatoid Arthritis patients. | | | | |
|---|---|---|---|---|
| Number of Samples | Subject assay | ENI assay | Abbott assay | Diagnosis: Sample I.D. |
| 6 | (+) | NT | (+) | NPC/NON-AIDS |
| 17 | - | NT | (+) | NPC/NON-AIDS |
| 8 | - | NT | - | NPC/NON-AIDS |
| 1 | - | NT | NT | NPC/NON-AIDS;920 |
| 1 | - | (+) | - | RA/NON-AIDS;305 |
| 7 | - | 2-,5NT | 3-,4NT | RA/NON-AIDS |
| 1 | - | - | (+) | RA/NON-AIDS;615 |
| (+) = False Positives NT = NOT TESTED NPC = Naso-Pharyngeal Carcinoma; RA = Rheumatoid Arthritis | | | | |

C. To indicate the effectiveness of the subject assay for detection of HTLV-III antibody in AIDS/ARC patient sera compared to commercial kits, the ELISA kit described in Example IV-Procedure 1 was evaluated against a panel of sera derived from diagnosed AIDS and ARC patients. The results are summarized in Table III and show that the assay is equivalent to commercial kits for its ability to identify sera containing antibody to HTLV-III.

TABLE III

| Assay of AIDS/ ARC sera: | | | | |
|---|---|---|---|---|
| Number of Samples | Subject assay | ENI assay | Abbott assay | Diagnosis/Sample I.D. |
| 67 | + | + | + (16NT) | AIDS |
| 2 | (-) | (-) | (-) | AIDS/533,3621 |
| 1 | (-) | + | + | AIDS/653 |
| 2 | + | + | (-) | AIDS/661,662 |
| 21 | + | + | + | ARC |
| 2 | (-) | (-) | (-) | ARC/512,529 |
| $\overline{95}$ | | | | |
| (-) = False Negatives<br>ARC = Aids Related Complex | | | | |

D. The high rate of false positives characteristic of presently available kits using viral lysate is due in part to the presence of cellular antigens in the lysate that react with antibodies present in both AIDS and non-AIDS patient sera. Additionally, complex antigens such as those derived from a virus such as HTLV-III contain many epitopes and are more likely to react non-specifically with antibodies present in human sera. The subject peptide (II) reduces the complexity of the antigen used to react with patient sera down to one or perhaps only a few epitopes. The chance of non-specific interaction with non-HTLV-III antibodies is expected to be greatly reduced. Non-specific interactions, however, may still occur since some antibodies are "sticky" and can bind to the plastic support used in the assay, or to other proteins such as bovine serum albumin or goat sera used to block the plate after addition of the peptide. Presented in Table IV below are data relating to the assay of various patient sera. In addition to the usual assay with peptide (II) as described in Example IV-Procedure 1 we have assayed each sera against peptide (II) after mixing said sera samples with an effective blocking amount of the peptide (II). Also included in the Table are the results of assaying each sample with commercially available kits.

TABLE IV

| Competition Assay Confirming Positive/Negative ELISA: | | | | | | |
|---|---|---|---|---|---|---|
| Samp. I.D. | ELISA Value & Score with Peptide | | Abbott Assay | ENI Assay | Western Assay | Diag. |
| | Not Blocked | Blocked (Score) | | | | |
| 615 | 0.031 | 0.033(-) | - | - | - | RA |
| 3195 | 0.026 | 0.045(-) | - | +* | - | DP |
| 3196 | 0.028 | 0.039(-) | - | +* | - | DP |
| 3197 | 0.065 | 0.073(-) | - | +* | - | DP |
| 3376 | 0.104 | 0.105(-) | - | +* | - | UNK |
| 3362 | 0.740 | 0.727(-) | - | +* | - | UNK |
| 912 | 0.055 | 0.065(-) | +* | NT | - | NPC |
| * = False Positive | | | | | | |

TABLE IV   (continued)

| Competition Assay Confirming Positive/Negative ELISA: | | | | | | |
|---|---|---|---|---|---|---|
| Samp. I.D. | ELISA Value & Score with Peptide | | Abbott Assay | ENI Assay | Western Assay | Diag. |
| | Not Blocked | Blocked (Score) | | | | |
| 918 | 0.100 | 0.092(-) | +* | NT | - | NPC |
| 922 | 0.127 | 0.103(-) | +* | NT | NT | NPC |
| 923 | 0.114 | 0.080(-) | +* | NT | NT | NPC |
| 3644 | 0.336 | 0.380(-) | +* | +* | - | UNK |
| 3406 | 1.400 | 1.390(-) | +* | +* | - | DP |
| 3461 | 1.426 | 1.137(-) | +* | +* | - | DP |
| 3532 | 1.770 | 0.080(+) | + | + | + | UNK |
| 3469 | 0.300 | 0.030(+) | + | + | + | UNK |
| 3431 | 0.507 | 0.087(+) | NT | + | + | UNK |
| 644 | 0.160 | 0.024(+) | NT | + | + | AIDS |
| 659 | 0.510 | 0.042(+) | + | + | + | AIDS |
| 661 | 0.500 | 0.031(+) | -** | + | + | AIDS |
| 662 | 0.160 | 0.030(+) | -** | + | + | AIDS |

* = False Positive

** = False Negative DP = Dialysis Patient, Non-Aids NPC = Naso-Pharyngeal Carcinoma, Non-Aids UNK = Unknown RA = Rheumatoid Arthritis, Non-Aids

It is evident from these results that non-AIDS sera samples incorrectly identified as positive by either or both of the commercially available kits are correctly identified as negative using the peptide competition assay. Furthermore, even samples incorrectly identified as positive by the assay are correctly identified as negative by the peptide competition assay. Significantly, the blocking or competition assay also serves as a confirmatory assay in tests of sera samples that do contain antibodies to HTLV-III. The last seven samples tested as shown in Table IV are positive for antibody by both the subject test and commercially available assays (with the exception of two false negatives using the Abbott kit) and by Western blot assay. The reactivity of these sera with peptide (II) is effectively blocked by mixing the sera with peptide (II) indicating that the reactivity of antibody to peptide is peptide specific and that these last seven samples are true positives.

EXAMPLE VI

ELISA kits as described in Example IV-Procedure 1 were made with peptide formulas (I) through (XV) as well as formulas (C-I) through (C-VIII). The ELISA kits were each evaluated against a ten sample panel of sera comprising clinically positive samples, i.e., samples which were symptomatic of the HTLV-III infection as determined by commercial assays and western blot assay. The results of these tests are shown in Table V below.

A peptide assay is considered positive if the absorbent level of the ELISA test was more than twice the background level as determined by averaging the absorbence of two normal sera. The mean reported represents the mean optical density level of the ELISA as calculated after the background level was subtracted. The index reported is a weighted activity of a given peptide relative to the activity of the peptide of formula (II). The index is weighted in favor of the ability of a particular peptide assay to correctly report a positive value as distinguished from the level of the background normal response. The formula for deriving the index is as follows:

$$\frac{(\% \text{ Positive})^3 \times \sqrt{\text{MEAN}}}{(\% \text{ Positive})_{II}^3 \times \sqrt{\text{MEAN}_{II}}} = \text{Index}$$

wherein:

% Positive is % Positive for the given peptide assay;

MEAN is MEAN for the given peptide assay; % Positive$_{II}$ is % Positive for peptide assay having the Formula (II);

and % MEAN$_{II}$ is MEAN for the peptide assay having the Formula (II).

**TABLE V**

| PEPTIDE FORMULA | SEQUENCE | %POSITIVE | MEAN | INDEX |
|---|---|---|---|---|
| (II) | IWGCSGKLICTTAVP | 80 | 1.67+0.82 | 1.00 |
| (C-V) | QLTVWGIKQLQARIL | 0 | – – | 0 |
| (C-VI) | GIKQLQARILAVERY | 20 | 0.45+0.06 | 0.01 |
| (C-I) | QLQARILAVERY | 0 | – – | 0 |
| (C-IX) | QARILAVERYLKDQQ | 0 | – – | 0 |
| (XV) | RILAVERYLKDQQLLGIWGCS | 90 | 2.01+1.40 | 1.56 |
| (C-II) | AVERYLKDQQLLG | 10 | 0.04 – | <0.01 |
| (C-VII) | AVERYLKDQQLLGIW | 60 | 1.18+0.68 | 0.35 |
| (IV) | AVERYLKDQQLLGIWGCSGKLI | 100 | 2.23+0.68 | 2.26 |
| (XII) | AVERYLKDQQLLGIWGCSGKLIC | 100 | 1.95+0.27 | 2.11 |
| (C-III) | LKDQQLLGIWGCS | 30 | 0.28+0.06 | 0.02 |
| (XIV) | LKDQQLLGIWGCSGK | 90 | 1.69+0.65 | 1.43 |
| (VI) | LKDQQLLGIWGCSGKLI | 100 | 1.19+0.85 | 1.65 |
| (VII) | LLGIWGCSGKLIC | 50 | 0.22+0.05 | 0.09 |
| (XI) | LLGIWGCSGKLICTT | 80 | 1.53+0.77 | 0.96 |
| (C-IV) | IWGCSGKLI | 20 | 0.17+0.00 | 0.01 |
| (I) | CSGKLIC | 60 | 0.34+0.11 | 0.19 |
| (VIII) | QQLLGIWGCSGKLICTTAVPWNAS | 90 | 0.51+0.25 | 0.79 |
| (IX) | IWGCSGKLICTTAVPWN | 70 | 0.86+0.45 | 0.48 |
| (III) | IWGCSGKLICTTAVPWNAS | 100 | 2.20+0.86 | 2.24 |
| (XIII) | GCSGKLICTTAVPWN | 100 | 2.14+0.58 | 2.21 |
| (X) | CSGKLICTTAVPWNAS | 100 | 1.74+1.01 | 1.99 |
| (XI) | SGKLICTTAVPWNAS | 50 | 0.86+0.63 | 0.17 |
| (C-VIII) | LICTTAVPWNASWSN | 0 | – – | 0 |
| (V) | AVERYLKDQQLLGIWGCSGKLICTTAVPWNAS | 100 | 1.53+0.66 | 1.89 |
| (III) & (IV) | AVERYLKDQQLLGIWGCSGKLI + IWGCSGKLICTTAVPWNAS | 100 | >3.0 – | 2.62 |

As can be seen from the above Table, assays made from peptides having the Formulas (I) through (XV), all provide positive results of at least 50% or greater and in each case manifest an index of at least 0.1. On the other hand, each of the comparative segments, although representing closely adjacent or overlapping or partially overlapping segments from the HTLV-III envelope, fail to exhibit such positive results or such high index.

EXAMPLE VII

Additional AIDS/ARC patient sera were tested with ELISA assays employing the two highly reactive peptides of formulas (III) and (IV) (Example IV-Procedure 1). The reactivity, expressed as absorbence values, of some of these sera is shown in Table VI.

TABLE VI

|  | SERUM SAMPLE | Peptide Formula | |
|---|---|---|---|
|  |  | (III) | (IV) |
| A. | 3362 | 0.503 | 0.151 |
|  | 3412 | >2.00 | 0.540 |
|  | 3693 | 0.559 | 0.120 |
|  | 3722 | 0.620 | 0.193 |
|  | 0649 | 1.756 | 0.379 |
| B. | 3544 | 0.428 | >2.00 |
|  | 3575 | 0.350 | 1.773 |
|  | 3744 | 0.311 | 1.326 |
|  | 3790 | 0.224 | 1.765 |
|  | 0509 | 0.403 | 2.000 |
|  | 0653 | 0.111 | 0.999 |
|  | 0662 | 0.301 | 1.392 |
| C. | 3416 | 1.914 | >2.00 |
|  | 3456 | 1.670 | 1.780 |
|  | 3666 | >2.00 | >2.00 |
|  | 3414 | 1.453 | 1.057 |
|  | 3411 | >2.00 | >2.00 |
|  | 3413 | >2.00 | >2.00 |

Most sera which were tested reacted very well with both peptides, much as is seen with samples in Table VI, group C. Occassionally, however, some samples were far more reactive with one peptide than the other, as shown in Table VI, groups A and B. These data indicate that there may be more than one epitope (e.g. a linear and a conformational epitope) in this thirty-two amino acid region that is commonly recognized by patients that have been exposed to AID virus. High performance liquid chromatography (HPLC) analysis of peptides of formulas (III) and (IV) in solution indicate that formula (III) peptides exist largely as cyclic monomers, while formula (IV) peptides are mostly in dimer form. The structural characteristics imparted to these two peptides by disulfide bonding may relate to both antigen presentation and the creation of a conformational epitope.

EXAMPLE VIII

Further evidence that more than one epitope is present in formula (III) and (IV) peptides can be deduced from competition studies, the results of an example of which are shown in Table VIIA. In this study, formula (III) and (IV) peptides were both applied to a microtiter plate as immobilized antigen and an ELISA assay was performed under five different conditions: without competition, or competition with excess formula (III) peptide, formula (IV), both peptides, or an heterologous peptide. The competition was performed by adding the appropriate peptide(s) to the diluted serum just before adding the serum sample to the microtiter well.

TABLE VIIA

| SAMPLE | UNBLOCKED | COMPETING PEPTIDE FORMULA | | | |
|---|---|---|---|---|---|
|  |  | (III) | (IV) | (III)/(IV) | HETEROLOGOUS |
| 3412 | 1.450 | 0.113 | 1.182 | 0.059 | 1.348 |
| 3413 | >2.00 | 0.381 | 2.032 | 0.068 | 2.026 |
| 3416 | >2.00 | 1.011 | 1.055 | 0.197 | 2.041 |
| 3544 | 1.810 | 0.876 | 0.137 | 0.048 | 1.750 |
| 3575 | 1.267 | 0.982 | 0.105 | 0.036 | 1.304 |
| 3693 | 0.340 | 0.103 | 0.217 | 0.065 | 0.293 |
| 3790 | 1.558 | 1.320 | 0.635 | 0.134 | 1.349 |

Under these conditions it is very clear that some samples react very well with each or both of the subject peptides. For example, sample 3416 reacts well with both peptides, since competition with the formula (III) peptide gives an optical density (OD) of >1.8, competition with the formula IV peptide yields an OD of 1.055 and, in the presence of both competing peptides, the OD goes down essentially to background levels. Other serum samples such as 3412 react much more strongly with one peptide than the other; in this case, the OD is reduced from >1.4 down to 0.113 when blocked with formula (III) peptide and remains >1 when competed with formula (IV) peptide. However, there is clearly some reactivity with formula (III) peptide because, in the presence of both peptides, immunoreactivity is completely abolished (0.059).

Another competition study with the formula (I) peptide on the plate, shows that formula (IV) peptide does not effectively compete with formula (I) peptide although formula (III) peptide competes very effectively. See Table VIIB below.

TABLE VIIB

| SAMPLE | UNBLOCKED | COMPETING PEPTIDE FORMULA | | | |
|---|---|---|---|---|---|
| | | (I) | (IV) | (III)/(IV) | HETEROLOGOUS |
| 013 | 1.177 | 0.038 | 0.046 | 0.770 | 0.865 |
| 014 | 0.383 | 0.050 | 0.067 | 0.273 | 0.324 |

Thus, it appears from these analyses that an epitope present in formula (III) peptide is also present on formula (I) peptide, and that this epitope is substantially different from those present in formula (IV) peptide. Furthermore, based on this same sample, virtually all sera react with the epitopes presented on the formula (III) and (IV) peptides, although in many cases, more strongly with one peptide than with the other.

EXAMPLE IX

Using 1µg of combination of formula (III) and 0.5 µg of formula (IV) peptides as the solid phase antigen in an ELISA assay (Example IV-Procedure 2), the specificity and sensitivity of this assay was equal or superior to any of the commercially available viral lysate antibody detection kits tested.

Table VIII presents ELISA results obtained using patient sera, normal sera, and sera from miscellaneous disease groups that include rheumatoid arthritis, naso-pharyngeal carcinoma (NPC), Epstein-Barr virus infection, cytomegalovirus infection, gram negative sepsis, toxoplasma gondii, systemic lupus erythematosus, and herpes virus infections.

TABLE VIII

| SAMPLE GROUP | NUMBER OF SERA | ENI+ | (III)/(IV)+ | ENI- | (III)/(IV)- |
|---|---|---|---|---|---|
| AIDS + ARC | 458(243)* | 449 | 450 | 9 | 8 |
| SYPTOMATIC PLS | 320(146) | 239 | 242 | 81 | 78 |
| ASYMPTOMATIC/HIGH RISK IMMUNE ABNORMALITIES | 135(87) | 38 | 39 | 97 | 96 |
| ASYMPTOMATIC/HIGH RISK IMMUNE NORMAL | 134(69) | 10 | 10 | 124 | 124 |
| NORMAL/NON-AIDS | 728(728) | 12 | 4 | 716 | 724 |
| MISCELLANEOUS DISEASE GROUPS/ NON-AIDS | 387(387) | 10 | 7 | 377 | 380 |
| TOTAL NON-AIDS | 1115 | 22 | 11 | 1093 | 1104 |

*Indicates number of patients.

When bona fide normal sera were tested for reactivity in the (III)/(IV) assay and in the assay marketed by Electronucleonics, Incorporated (ENI), the (III)/(IV) peptide assay has a significantly lower false positive rate. As Table VIII shows, the false positive rate for ENI was 1.65% (12/728), while the (III)/(IV) peptide assay has a false positive rate of only 0.55% (4/728). When the false positive rate in the Miscellaneous Disease Group is examined, the peptide assay had a slightly lower false positive rate than did the ENI assay. 1.01% vs. 2.58% (7/387 vs. 10/387).

The above examples have been given only for illustration purposes and not to limit the scope of the present invention which scope is defined only in the appended claims.

**Claims**

1. A synthetic peptide having one of the following formulae:

$$IWGCSGKLICTTAVP \qquad (II)$$

$$IWGCSGKLICTTAVPWNAS \qquad (III)$$

$$AVERYLKDQQLLGIWGCSGKLI \qquad (IV)$$

$$AVERYLKDQQLLGIWGCSGKLICTTAVPWNAS \qquad (V)$$

$$LKDQQLLGIWGCSGKLI \qquad (VI)$$

$$QQLLGIWGCSGKLICTTAVPWNAS \qquad (VIII)$$

$$CSGKLICTTAVPWNAS \qquad (X)$$

$$AVERYLKDQQLLGIWGCSGKLIC \qquad (XII)$$

$$GCSGKLICTTAVPWN \qquad (XIII)$$

$$LKDQQLLGIWGCSGK \qquad (XIV)$$

2. The peptide of claim 1 in cyclic monomeric, dimeric or polymeric form.

3. A mixture of at least a first and a second peptide, wherein:

said mixture has enhanced recognition for antibodies to HTLV-III virus as compared to each peptide taken alone;
said first peptide is one of the following peptides

$$CSGKLIC \qquad (I)$$

IWGCSGKLICTTAVP (II)

IWGCSGKLICTTAVPWNAS (III)

AVERYLKDQQLLGIWGCSGKLICTTAVPWNAS (V)

LLGIWGCSGKLIC (VII)

QQLLGIWGCSGKLICTTAVPWNAS (VIII)

IWGCSGKLICTTAVPWN (IX)

CSGKLICTTAVPWNAS (X)

AVERYLKDQQLLGIWGCSGKLIC (XII)

GCSGKLICTTAVPWN (XIII);

said second peptide is one of the following peptides

AVERYLKDQQLLGIWGCSGKLI (IV)

AVERYLKDQQLLGIWGCSGKLICTTAVPWNAS (V)

LKDQQLLGIWGCSGKLI (VI)

QQLLGIWGCSGKLICTTAVPWNAS (VIII)

AVERYLKDQQLLGIWGCSGKLIC (XII)

LKDQQLLGIWGCSGK (XIV)

## RILAVERYLKDQQLLGIWGCS (XV)

or any one of those peptides in which the I residue in the subsequence LLGIW has been substituted by a L, M or F residue, provided that the first and second peptides are different.

4. The mixture of claim 3 wherein said first peptide is one of the peptide III, X and XIII as defined in claim 3; and

said second peptide is either peptide IV or peptide XII as defined in claim 3 or either of these peptides in which the I residue in the subsequence LLGIW has been substituted by a L, M or F residue.

5. The mixture of claim 4 wherein the first peptide is peptide III and the second peptide is peptide IV or peptide IV in which the I residue in the subsequence LLGIW has been substituted by a L, M or F residue.

6. The mixture of claim 4 wherein the first peptide is peptide XIII and the second peptide is peptide IV or peptide IV in which the I residue in the subsequence LLGIW has been substituted by a L, M or F residue.

7. The mixture of claim 5 or claim 6 wherein the second peptide is peptide IV.

8. A method, for detecting or determining antibodies to HTLV-III in a fluid suspected of containing said antibody, which comprises:

a) providing a peptide of claim 1 or claim 2 attached to a solid surface;
b) contacting said peptide-coated solid surface with the fluid to be tested for a sufficient time to allow an immunologic reaction to occur; and
c) detecting or determining the presence or amount of antibodies bound to said peptide on the surface of said solid.

9. The method of claim 8 wherein the detecting or determining step comprises contacting said surface with labelled anti-human immunoglobulin.

10. A method of preparing antibodies to HTLV-III virus which comprises: immunizing an animal with an immunogenically-effective amount of a peptide of claim 1 or claim 2, in polymerized form and/or attached to a suitable immunogenic carrier; and bleeding said animal.

11. A diagnostic kit for detecting or determining antibodies to HTLV-III which comprises:

(a) a solid surface having bound thereto a peptide of claim 1 or claim 2; and
(b) labelled anti-human immunoglobulins.

12. A sandwich method, for detecting or determining HTLV-III virus or viral-specific antigen in a fluid suspected of containing said virus or antigen, which comprises:

a) providing antibody to a peptide of claim 1 or claim 2 attached to a solid surface;
b) contacting said antibody-coated solid surface with the fluid to be tested for a sufficient time to allow an immunologic reaction to occur; and
c) detecting or determining the presence or amount of HTLV-III virus or viral specific antigen attached to said antibodies on said solid surface.

13. A competition method, for determining HTLV-III virus or viral specific antigen in a fluid suspected of containing said virus or antigen, comprising the steps of:

a) providing an antibody to a peptide of claim 1 or claim 2, said antibody being attached to a solid surface;
b) mixing an aliquot of the fluid to be tested with a known amount of labelled subject peptide to produce a mixed sample;
c) contacting said antibody - coated solid surface with said mixed sample for a sufficient time to allow an immunologic reaction to occur;

d) separating the solid surface from the mixed sample;

e) detecting or determining the presence or amount of labelled peptide either bound to the solid surface or remaining in the mixed sample; and

f) determining from the result in step e) the presence or amount of said virus or antigen in said fluid.

**14.** A diagnostic kit for detecting or determining HTLV-III virus or viral specific antigen, which comprises:

a) a solid surface having bound thereto antibody to a peptide of claim 1 or claim 2; and

b) a known amount of labelled antibody to HTLV-III, to a viral specific antigen, or to a peptide of claim 1 or claim 2.

**15.** A method, for determining the accuracy of a positive result in a test for HTLV-III antibodies on a fluid sample suspected of containing said antibodies, said test being a sandwich assay in which a synthetic HTLV-III peptide according to claim 1 or claim 2 is attached to the solid surface, said method comprising the steps of:

a) mixing an aliquot of said sample with an effective blocking amount of said peptide to produce a mixed sample;

b) contacting the mixed sample with the solid phase of said test for a sufficient time to allow an immunlogic reaction to occur;

c) determining whether binding of said antibodies in said mixed sample with said solid phase is inhibited compared to said binding in the absence of said peptide; and

d) determining, based on the presence or absence of said inhibition, the accuracy of said positive result.

**16.** A diagnostic kit, for determining or detecting antibody to HTLV-III and confirming a positive result, which comprises:

a) a solid surface having bound thereto a peptide of claim 1 or claim 2;

b) an effective blocking amount of the said peptide; and

c) labelled anti-human immunoglobulin.

**17.** A method, for preparing monoclonal antibodies to HTL V-III, comprising:

a) immunizing a host animal with a peptide of claim 1 or claim 2 in polymerized form or attached to a suitable immunogenic carrier;

b) isolating splenocytes from said immunized host;

c) fusing said splenocytes with a suitable myeloma cell line;

d) selecting the fused cells by reactivity to the immunizing peptide, to HTLV-III, or to HTLV-III-infected cells;

e) either culturing the selected hybridoma in vitro or injecting it into a suitable host; and

f) recovering the desired monoclonal antibody from the supernatant over the cultured hybridoma or from the serum or ascites of the injected host.

**Patentansprüche**

**1.** Synthetisches Peptid mit einer der folgenden Formeln:

$$\text{IWGCSGKLICTTAVP} \qquad \text{(II)}$$

$$\text{IWGCSGKLICTTAVPWNAS} \qquad \text{(III)}$$

$$\text{AVERYLKDQQLLGIWGCSGKLI} \qquad \text{(IV)}$$

$$\text{AVERYLKDQQLLGIWGCSGKLICTTAVPWNAS} \qquad \text{(V)}$$

LKDQQLLGIWGCSGKLI (VI)

QQLLGIWGCSGKLICTTAVPWNAS (VIII)

CSGKLICTTAVPWNAS (X)

AVERYLKDQQLLGIWGCSGKLIC (XII)

GCSGKLICTTAVPWN (XIII)

LKDQQLLGIWGCSGK (XIV)

**2.** Peptid nach Anspruch 1 in zyklischer, monomerer, dimerer oder polymerer Form.

**3.** Mischung aus wenigstens einem ersten und einem zweiten Peptid, worin:

die Mischung im Vergleich zu jedem einzeln verwendeten Peptid von gegen HTLV-III-Virus gerichteten Antikörpern besser erkannt wird;

das erste Peptid eines der folgenden Peptide ist

CSGKLIC (I)

IWGCSGKLICTTAVP (II)

IWGCSGKLICTTAVPWNAS (III)

AVERYLKDQQLLGIWGCSGKLICTTAVPWNAS (V)

LLGIWGCSGKLIC (VII)

QQLLGIWGCSGKLICTTAVPWNAS (VIII)

IWGCSGKLICTTAVPWN (IX)

CSGKLICTTAVPWNAS (X)

AVERYLKDQQLLGIWGCSGKLIC (XII)

GCSGKLICTTAVPWN (XIII);

das zweite Peptid eines der folgenden Peptide

AVERYLKDQQLLGIWGCSGKLI (IV)

AVERYLKDQQLLGIWGCSGKLICTTAVPWNAS (V)

LKDQQLLGIWGCSGKLI (VI)

QQLLGIWGCSGKLICTTAVPWNAS (VIII)

AVERYLKDQQLLGIWGCSGKLIC (XII)

LKDQQLLGIWGCSGK (XIV)

RILAVERYLKDQQLLGIWGCS (XV)

oder eines von diesen Peptiden, in welchem der Rest I in der Teilsequenz LLGIW durch einen Rest L, M oder F ersetzt worden ist, ist, mit der Maßgabe, dass die ersten und zweiten Peptide verschieden sind.

4. Mischung nach Anspruch 3, worin das erste Peptid eines der Peptide III, X und XIII ist, die in Anspruch 3 definiert sind; und

das zweite Peptid entweder Peptid IV oder Peptid XII, die in Anspruch 3 definiert sind, oder eines dieser Peptide, in welchem der Rest I in der Teilsequenz LLGIW durch einen Rest L, M oder F ersetzt worden ist, ist.

5. Mischung nach Anspruch 4, worin das erste Peptid Peptid III ist und das zweite Peptid Peptid IV oder Peptid IV, in welchem der Rest I in der Teilsequenz LLGIW durch einen Rest L, M oder F ersetzt worden ist, ist.

6. Mischung nach Anspruch 4, worin das erste Peptid Peptid XIII ist und das zweite Peptid Peptid IV oder Peptid IV, in welchem der Rest I in der Teilsequenz LLGIW durch einen Rest L, M oder F ersetzt worden ist, ist.

7. Mischung nach Anspruch 5 oder Anspruch 6, worin das zweite Peptid Peptid IV ist.

8. Verfahren zum Nachweisen oder Bestimmen von gegen HTLV-III gerichteten Antikörpern in einer Flüssigkeit, die vermutlich diesen Antikörper enthält, welches umfasst:

a) Bereitstellen eines an eine feste Oberfläche angehefteten Peptids nach Anspruch 1 oder Anspruch 2;

b) Inkontaktbringen dieser mit einem Peptid beschichteten festen Oberfläche mit der zu analysierenden Flüssigkeit für eine Zeitdauer, die ausreichend ist, dass eine immunologische Reaktion stattfinden kann; und

c) Nachweisen oder Bestimmen des Vorhandenseins oder der Menge an Antikörpern, die auf der Oberfläche des Festkörpers an das Peptid gebunden sind.

9. Verfahren nach Anspruch 8, worin das Nachweisen oder Bestimmen den Schritt des Inkontaktbringens der Oberfläche mit markiertem Anti-Human-Immunglobulin umfasst.

**10.** Verfahren zum Herstellen von gegen das HTLV-III-Virus gerichteten Antikörpern, welches das Immunisieren eines Tieres mit einer zum Erzeugen von Immunität wirksamen Menge eines Peptids nach Anspruch 1 oder Anspruch 2, in polymerisierter Form und/oder an einen geeigneten immunogenen Träger angeheftet, und die Entnahme von Blut von diesem Tier umfasst.

**11.** Diagnostischer Kit zum Nachweisen oder Bestimmen von gegen HTLV-III gerichteten Antikörpern, welcher umfasst:

(a) eine feste Oberfläche, an die ein Peptid nach Anspruch 1 oder Anspruch 2 gebunden ist; und

(b) markierte Anti-Human-Immunglobuline.

**12.** Sandwich-Verfahren zum Nachweisen oder Bestimmen des HTLV-III-Virus oder eines Virus-spezifischen Antigens in einer Flüssigkeit, die vermutlich das Virus oder Antigen enthält, welches umfasst:

a) Bereitstellen eines gegen ein Peptid nach Anspruch 1 oder Anspruch 2 gerichteten Antikörpers, der an eine feste Oberfläche angeheftet ist;

b) Inkontaktbringen der mit dem Antikörper beschichteten festen Oberfläche mit der zu analysierenden Flüssigkeit für eine Zeitdauer, die ausreichend ist, dass eine immunologische Reaktion stattfinden kann; und

c) Nachweisen oder Bestimmen des Vorhandenseins oder der Menge an HTLV-III-Virus oder des Virus-spezifischen Antigens, das an die Antikörper auf der festen Oberfläche angeheftet ist.

**13.** Kompetitionsverfahren zum Bestimmen von HTLV-III-Virus oder Virusspezifischem Antigen in einer Flüssigkeit, die vermutlich das Virus oder Antigen enthält, umfassend die Schritte:

a) Bereitstellen eines gegen ein Peptid nach Anspruch 1 oder Anspruch 2 gerichteten Antikörpers, wobei der Antikörper an eine feste Oberfläche angeheftet ist;

b) Mischen eines Aliquots der zu analysierenden Flüssigkeit mit einer bekannten Menge des markierten erfindungsgemäßen Peptids, um ein Probengemisch herzustellen;

c) Inkontaktbringen der mit dem Antikörper beschichteten festen Oberfläche mit dem Probengemisch für eine Zeitdauer, die ausreichend ist, dass eine immunologische Reaktion stattfinden kann;

d) Trennen der festen Oberfläche von dem Probengemisch;

e) Nachweisen oder Bestimmen des Vorhandenseins oder der Menge an markiertem Peptid, das entweder an die feste Oberfläche gebunden ist oder in dem Probengemisch verbleibt; und

f) Bestimmen des Vorhandenseins oder der Menge des Virus oder Antigens in der Flüssigkeit durch das Ergebnis in Stufe e).

**14.** Diagnostischer Kit zum Nachweisen oder Bestimmen von HTLV-III-Virus oder Virus-spezifischem Antigen, welcher umfasst:

a) eine feste Oberfläche, an die ein gegen ein Peptid nach Anspruch 1 oder Anspruch 2 gerichteter Antikörper gebunden ist; und

b) eine bekannte Menge eines gegen HTLV-III, gegen ein Vrus-spezifisches Antigen oder gegen ein Peptid nach Anspruch 1 oder Anspruch 2 gerichteten markierten Antikörpers.

**15.** Verfahren zum Bestimmen der Genauigkeit eines positiven Ergebnisses, das in einem Test auf gegen HTLV-III gerichtete Antikörper erhalten wird, ausgeführt mit einer flüssigen Analysenprobe, die vermutlich die Antikörper enthält, wobei der Test ein Sandwich-Assay ist, bei dem ein synthetisches HTLV-III-Peptid nach Anspruch 1 oder Anspruch 2 an die feste Oberfläche angeheftet ist, wobei das Verfahren die Schritte umfasst:

a) Mischen eines Aliquots der Analysenprobe mit einer wirksamen Blockierungsmenge des Peptids, um ein Probengemisch herzustellen;

b) Inkontaktbringen des Probengemisches mit der festen Phase des Tests für eine Zeitdauer, die ausreichend ist, dass eine immunologische Reaktion stattfinden kann;

c) Bestimmen, ob das Binden der Antikörper im Probengemisch an die feste Phase gehemmt ist im Vergleich zum Binden in der Abwesenheit des Peptids; und

d) Bestimmen der Genauigkeit des positiven Ergebnisses auf der Grundlage des Vorhandenseins oder der Abwesenheit der Hemmung.

16. Diagnostischer Kit zum Bestimmen oder Nachweisen von gegen HTLV-III gerichtetem Antikörper und Bestätigen eines positiven Ergebnisses, welcher umfasst:

a) eine feste Oberfläche, an die ein Peptid nach Anspruch 1 oder Anspruch 2 gebunden ist;

b) eine wirksame Blockierungsmenge des Peptids; und

c) markiertes Anti-Human-Immunglobulin.

17. Verfahren zum Herstellen von gegen HTLV-III.gerichteten monoclonalen Antikörpern, umfassend:

a) Immunisieren eines Wirtstieres mit einem Peptid nach Anspruch 1 oder Anspruch 2 in polymerisierter Form oder gebunden an einen geeigneten immunogenen Träger;

b) Isolieren von Splenozyten aus dem immunisierten Wirt;

c) Fusionieren der Splenozyten mit einer geeigneten Myelom-Zelllinie;

d) Auswählen der fusionierten Zellen durch Reaktionsfähigkeit gegenüber dem immunisierenden Peptid, gegenüber HTLV-III oder gegenüber mit HTLV-III infizierten Zellen;

e) entweder Kultivieren des ausgewählten Hybridoms in vitro oder Injizieren desselben in einen geeigneten Wirt; und

f) Gewinnen des gewünschten monoclonalen Antikörpers aus dem Überstand über dem kultivierten Hybridom oder aus dem Serum oder Aszites des Wirts, in den injiziert wurde.

**Revendications**

1. Peptide synthétique possédant l'une des formules suivantes :

IWGCSGKLICTTAVP                    (II)

IWGCSGKLICTTAVPWNAS                (III)

AVERYLKDQQLLGIWGCSGKLI                  (IV)

AVERYLKDQQLLGIWGCSGKLICTTAVPWNAS        (V)

LKDQQLLGIWGCSGKLI (VI)

QQLLGIWGCSGKLICTTAVPWNAS (VIII)

CSGKLICTTAVPWNAS (X)

AVERYLKDQQLLGIWGCSGKLIC (XII)

GCSGKLICTTAVPWN (XIII)

LKDQQLLGIWGCSGK (XIV)

2. Peptide selon la revendication 1 sous forme cyclique monomère, dimère ou polymère.

3. Mélange d'au moins un premier et un second peptide, dans lequel :

ledit mélange présente une reconnaissance accrue pour les anticorps du virus HTLV-III par rapport à chaque peptide pris isolément ;
ledit premier peptide est un des peptides suivants

CSGKLIC (I)

IWGCSGKLICTTAVP (II)

IWGCSGKLICTTAVPWNAS (III)

AVERYLKDQQLLGIWGCSGKLICTTAVPWNAS (V)

LLGIWGCSGKLIC (VII)

QQLLGIWGCSGKLICTTAVPWNAS (VIII)

IWGCSGKLICTTAVPWN (IX)

CSGKLICTTAVPWNAS (X)

AVERYLKDQQLLGIWGCSGKLIC (XII)

GCSGKLICTTAVPWN (XIII)

ledit second peptide est un des peptides suivants

AVERYLKDQQLLGIWGCSGKLI                    (IV)

AVERYLKDQQLLGIWGCSGKLICTTAVPWNAS  (V)

LKDQQLLGIWGCSGKLI                    (VI)

QQLLGIWGCSGKLICTTAVPWNAS  (VIII)

AVERYLKDQQLLGIWGCSGKLIC            (XII)

LKDQQLLGIWGCSGK                    (XIV)

RILAVERYLKDQQLLGIWGCS            (XV)

ou un quelconque de ces peptides dans lequel le résidu I dans la sous-séquence LLGIW a été substitué par un résidu L, M ou F, à condition que les premier et second peptides soient différents.

4. Mélange selon la revendication 3 dans lequel ledit premier peptide est un des peptides III, X et XIII tels que définis dans la revendication 3 ; et

ledit second peptide est soit le peptide IV, soit le peptide XII, tels que définis dans la revendication 3, ou bien l'un ou l'autre de ces peptides dans lesquels le résidu I dans la sous-séquence LLGIW a été substitué par un résidu L, M ou F.

5. Mélange selon la revendication 4 dans lequel le premier peptide est le peptide III et le second peptide est le peptide IV ou le peptide IV dans lequel le résidu I dans la sous-séquence LLGIW a été substitué par un résidu L, M ou F.

6. Mélange selon la revendication 4 dans lequel le premier peptide est le peptide XIII et le second peptide est le peptide IV ou le peptide IV dans lequel le résidu I dans la sous-séquence LLGIW a été substitué par un résidu L, M ou F.

7. Mélange selon la revendication 5 ou la revendication 6 dans lequel le second peptide est le peptide IV.

8. Procédé pour détecter ou déterminer des anticorps contre HTLV-III dans un fluide soupçonné de contenir ledit anticorps, qui comprend les étapes consistant à :

a) disposer d'un peptide selon la revendication 1 ou la revendication 2 fixé à une surface solide ;
b) mettre en contact ladite surface solide revêtue de peptide avec le fluide à tester pendant un temps suffisant pour qu'une réaction immunologique ait lieu ; et
c) détecter ou déterminer la présence ou la quantité d'anticorps liés audit peptide sur la surface dudit solide.

9. Procédé selon la revendication 8 dans lequel l'étape de détection ou de détermination comprend la mise en contact de ladite surface avec une anti-immunoglobuline humaine marquée.

10. Procédé de préparation d'anticorps contre le virus HTLV-III qui comprend : l'immunisation d'un animal avec une quantité efficace sur le plan immunogène d'un peptide selon la revendication 1 ou la revendication 2, sous une forme polymérisée et/ou fixé sur un véhicule immunogène approprié, et le saignement dudit animal.

11. Trousse de diagnostic pour détecter ou déterminer des anticorps contre HTLV-III, qui comprend :

(a) une surface solide sur laquelle est fixé un peptide selon la revendication 1 ou la revendication 2 ; et

(b) des anti-immunoglobulines humaines marquées.

**12.** Procédé en «sandwich» pour détecter ou déterminer le virus HTLV-III ou un antigène spécifique du virus dans un fluide soupçonné de contenir ledit virus ou ledit antigène, qui comprend les étapes consistant à :

a) disposer d'un anticorps contre un peptide selon la revendication 1 ou la revendication 2 fixé à une surface solide ;

b) mettre en contact ladite surface solide revêtue d'anticorps avec le fluide à tester pendant un temps suffisant pour qu'une réaction immunologique ait lieu ; et

c) détecter ou déterminer la présence ou la quantité de virus HTLV-III ou d'antigène spécifique du virus fixé auxdits anticorps sur ladite surface solide.

**13.** Procédé de compétition, pour déterminer le virus HTLV-III ou un antigène spécifique du virus dans un fluide soupçonné de contenir ledit virus ou ledit antigène, comprenant les étapes consistant à :

a) disposer d'un anticorps contre un peptide selon la revendication 1 ou la revendication 2, ledit anticorps étant fixé à une surface solide ;

b) mélanger une partie aliquote du fluide à tester avec une quantité connue du peptide marqué concerné pour produire un échantillon mélangé ;

c) mettre en contact ladite surface solide revêtue d'anticorps avec ledit échantillon mélangé pendant un temps suffisant pour qu'une réaction immunologique ait lieu ;

d) séparer la surface solide de l'échantillon mélangé ;

e) détecter ou déterminer la présence ou la quantité de peptide marqué, soit fixé à la surface solide ou restant dans l'échantillon mélangé ; et

f) déterminer, d'après le résultat de l'étape e), la présence ou la quantité dudit virus ou dudit antigène dans ledit fluide.

**14.** Trousse de diagnostic pour détecter ou déterminer le virus HTLV-III ou un antigène spécifique du virus, qui comprend:

(a) une surface solide sur laquelle est fixé un anticorps contre un peptide selon la revendication 1 ou la revendication 2 ; et

(b) une quantité connue d'anticorps marqué contre HTLV-III, contre un antigène spécifique du virus ou contre un peptide selon la revendication 1 ou la revendication 2.

**15.** Procédé pour déterminer l'exactitude d'un résultat positif dans un test d'anticorps contre HTLV-III sur un échantillon de fluide soupçonné de contenir lesdits anticorps, ledit test étant un test en «sandwich» dans lequel un peptide HTLV-III synthétique selon la revendication 1 ou la revendication 2 est fixé à la surface solide, ledit procédé comprenant les étapes consistant à :

a) mélanger une partie aliquote dudit échantillon avec une quantité bloquante efficace dudit peptide pour produire un échantillon mélangé ;

b) mettre en contact l'échantillon mélangé avec la phase solide dudit test pendant un temps suffisant pour qu'une réaction immunologique ait lieu ;

c) déterminer si la liaison desdits anticorps dans ledit échantillon mélangé avec ladite phase solide est inhibée par rapport à ladite liaison en l'absence dudit peptide ; et

d) déterminer, en se basant sur la présence ou l'absence de ladite inhibition, l'exactitude dudit résultat positif.

**16.** Trousse de diagnostic pour déterminer ou détecter un anticorps contre HTLV-III et confirmer un résultat positif, qui comprend :

a) une surface solide sur laquelle est fixé un peptide selon la revendication 1 ou la revendication 2 ;

b) une quantité bloquante efficace dudit peptide ; et

c) une anti-immunoglobuline humaine marquée.

**17.** Procédé de préparation d'anticorps monoclonaux contre HTLV-III, comprenant les étapes consistant à :

a) immuniser un animal hôte avec un peptide selon la revendication 1 ou la revendication 2 sous une forme polymérisée ou fixé à un véhicule immunogène approprié ;

b) isoler les splénocytes dudit hôte immunisé ;

c) fusionner lesdits splénocytes avec une lignée de cellules de myélome appropriée ;

d) sélectionner les cellules fusionnées par réactivité avec le peptide immunisant, avec HTLV-III ou avec les cellules infectées par HTLV-III ;

e) soit cultiver l'hybridome sélectionné in vitro ou l'injecter dans un hôte approprié ; et

f) récupérer l'anticorps monoclonal recherché dans le surnageant au-dessus de l'hybridome cultivé ou dans le sérum ou les ascites de l'hôte ayant reçu l'injection.